(19)

**Europäisches Patentamt**
**European Patent Office**
**Office européen des brevets**

(11) **EP 4 649 885 A1**

(12) **EUROPEAN PATENT APPLICATION**

(43) Date of publication:
**19.11.2025  Bulletin 2025/47**

(21) Application number: **25176864.4**

(22) Date of filing: **16.05.2025**

(51) International Patent Classification (IPC):
**A61B 5/02** (2006.01)     **A61B 5/0215** (2006.01)
**A61B 5/00** (2006.01)

(52) Cooperative Patent Classification (CPC):
**A61B 5/02007; A61B 5/0215; A61B 5/7239;**
**A61B 5/725;** A61B 5/6852

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **17.05.2024  PT 2024119467**

(71) Applicants:
• **Universidade de Évora**
  **7000-803 Évora (PT)**
• **Instituto Superior Técnico**
  **1049-001 Lisboa (PT)**

• **HOSPITAL DO ESPÍRITO SANTO DE ÉVORA,**
  **E.P.E**
  **7000-811 Evora (PT)**

(72) Inventors:
• **TIAGO, JORGE FILIPE DUARTE**
  **2855-083 CORROIOS (PT)**
• **NEVES, DAVID CINTRA HENRIQUES SILVA**
  **ÉVORA 7005-161 (PT)**
• **OLIVEIRA, MARÍLIA DA CONCEIÇÃO VALENTE**
  **DE**
  **2685-136 SACAVÉM (PT)**

(74) Representative: **Couto, Cláudia**
  **Clarke, Modet Portugal**
  **Av. Casal Ribeiro, 50 - 3º Dto.**
  **1000-093 Lisboa (PT)**

(54) **COMPUTER IMPLEMENTED METHOD FOR THE ASSESSMENT OF THE SEVERITY OF A CORONARY STENOSIS AT REST OR UNDER HYPEREMIA**

(57)     The present application relates to a computer implemented method for the assessment of the severity of a coronary stenosis. The method provides data values from the peak of the second derivative of a distal pressure curve which can be used as an indicator to assess the severity of a coronary stenosis in a subject in comparison with indicators from known tests. The data from a distal pressure curve used in the method of the present invention is acquired at rest or under hyperaemia conditions.

Figure 5

## Description

## Technical field

This application relates to the field of coronary artery diseases, and particularly relates to a computer implemented method for the assessment of the severity of coronary stenosis at rest or under hyperemia conditions.

## Background art

Due to the ageing of the population and their lifestyle habits (diet, sedentary lifestyle), cardiovascular risk factors such as hypertension, hyperglycaemia and excess adipose tissue are becoming more common in today's population. Coronary Artery Disease (CAD) is one of the pathologies that results from the interaction of these risk factors with blood cells and blood vessel wall and is characterized by inflammation and the accumulation of atherosclerotic plaque in coronary arteries, causing angina pectoris, myocardial infarction and ischemic heart failure [1, 2]. The treatment of CAD aims to alleviate angina symptoms and prevent acute myocardial infarction or premature death.

In order to suit the decision on whether to treat or not and to choose the most appropriate treatment method to apply in each case, it is crucial to have a diagnosis tool that could provide reliable information on the severity of each lesion. Therefore, several diagnosis tools are available in clinical practice, from non-invasive to invasive techniques as is the case of coronary angiography [3, 4].

The gold-standard test for physiological lesion assessment is Fractional Flow Reserve (FFR) [5,2]. However, the administration of vasodilating agents, such as adenosine, to induce maximal hyperaemia, needful to measure FFR, is the cause of several side effects [6]. These vasodilating agents minimize microvascular resistance and, thus, maximize blood flow across the stenosis. Healthy vessels and vessels with stenotic lesions respond differently to this hyperaemic stimulus. In the presence of a stenosis the vasodilating agent moderately increases coronary blood flow with a marked increase in the aortic-distal coronary pressure gradient [7].

Some alternatives to FFR have been proposed as is the case of the Instantaneous Wave Free Ratio (iFR) [8]. iFR is measured at rest avoiding the need of hyperaemia and, subsequently, of the administration of vasodilating agents.

Both FFR and iFR tests are based on pressure measurement, resulting in a calculation of the ratio of the downstream pressure - distal pressure (Pd) and upstream - aortic pressure (Pa) of the stenosis. While the FFR is determined during the injection of a vasodilator drug (hyperemia inducer), the iFR has the advantage of not requiring the administration of drugs, since it consists of software that allows analysing a specific part of the diastole, during which the curve of pressures presents a smooth behaviour (wave-free period). Thus, FFR consists of the continuous Pd/Pa ratio (of the entire cardiac cycle) under drug-induced hyperemia (usually adenosine). The iFR consists of the Pd/Pa ratio during a period of diastole, at rest (without administration of any drug). Both indicators show robust evidence of an advantage in their use compared to simple angiography. In addition to FFR and iFR, contrast hyperemia consists of an assessment of the Pd/Pa ratio in the period immediately after intracoronary injection of contrast, which causes a partial hyperemia, accentuating the turbulence effect caused by the stenosis.

However, iFR is commercially restricted to a proprietary software which limits its extended clinical application. The fact that the contrast agent normally used in clinical practice could induce submaximal hyperaemia, led to the development of an index, similar to FFR, that makes use of contrast medium instead of a vasodilating agent to induce hyperaemia, the Contrast Fractional Flow Reserve (cFFR) [9, 10]. There are many limitations to the use of cFFR, such as the absence of a dedicated software, the need to record a damped proximal pressure due to contrast injection and the lack of an established protocol for data acquisition.

The currently available tests and indicators described above still present several disadvantages. Even with these invasive tools, in a large percentage of cases intermediate values are obtained and the decision to revascularize or differ is difficult. The interpretation of the results of these tests is not always evident, especially when they present intermediate or incongruous values between tests. The sensors used are sensitive to handling, and in some cases, at the end of the procedure, when the measurements are validated by placing both sensors in the same position, it appears that they are not equalized - drift phenomenon-which implies the repetition of the measurements.

One of the main criticisms of iFR is the absence of hyperemic stimulus, which can cause a lower sensitivity compared to FFR, this phenomenon being particularly relevant in younger patients and with a large myocardial mass underlying the lesion evaluated. The contrast injection (necessarily performed on any coronary angiography) is, in itself, a hyperemic stimulus, which accentuates the pressure differences, especially when the lesion is significant - "contrast hyperemia". The assessment of pressures during contrast-induced hyperemia is thus positioned, from a physiological point of view, between (and complementarily) the iFR and the FFR, with regard to the vasodilator stimulus [3,4]. This technique is easy to use, innocuous, fast and already has some scientific evidence of practical use [11,5], with several studies in progress to optimize it. However, there is no software on the market dedicated to this technique, which is consequently done manually and with little standardization [2].

In US20030032886A, Dgany E et al describe a pressure-derived formula for estimating CFR, using the assumption of proportionality between pressure and flow. This method has the advantage of dismissing the need for doppler flow wires or thermodilution techniques. However, the limitations of the simplifying hypotheses on which it is based affect its reliability. Although it has been described for more than 15 years, the RFC has not gained much clinical relevance, probably due to both the inaccuracies observed in estimating the RFC and the reduced added value when compared to the RFF.

Document US20140276137A1, Burnett J et al also described a method for estimating CFR through pressure curve analysis.

This method is aimed at facilitating the estimation of stenosis severity, at the expense of many mathematical assumptions for the estimation of flow through pressure analysis. It is not used widely, also due to the lack of a clear benefice when compared to available indices.

Both these methods attempt to estimate flow by analysing pressure curves. Our method describes a different analysis of pressure curves that does not attempt to estimate flow, but rather describes the resistive effect of a given stenosis on a pressure curve, which implies the presence of ischaemia. It has the potential of an added value because it aims to tackle the same problem through a different pathway - curve shape analysis, rather than flow estimation.

**Summary**

The present invention relates to a computer implemented method for the assessment of the severity of a coronary stenosis according to the following steps:

- Acquiring an unfiltered distal pressure curve (unfPd) downstream of a stenosis previously identified in a subject;
- Filtering the unfiltered the distal pressure curve data (unfPd) by fitting a regular function to the data to obtain a filtered distal pressure curve (Pd);
- Identifying, in the previously filtered distal pressure curve (Pd), the cardiac cycles within a cardiac time period T, which is calculated as the average of the time intervals between consecutive systolic peaks;
- Calculating the first derivative of the pressure curve (dPd) and calculating for each cardiac cycle:

  - the minimum data value corresponding to a t_min time;
  - the maximum data value in a time interval [t_min, t_min+T];
  - the zero data value corresponding to the beginning of each cardiac cycle and located between the minimum and the maximum data values;
  - Mapping the data values in the distal pressure curve (Pd);

- Calculating the dicrotic notch for each cardiac cycle of the distal pressure curve (Pd) by using the first derivative of the pressure curve (dPd) to calculate:

  - The minimum data value along the first 50 to 70% time interval of each cardiac cycle;
  - The dicrotic notch as being the zero data value closest to zero pressure in the forthcoming 10 to 20% time interval of each cardiac cycle; or identifying the zero data values in the third derivative of the pressure curves (d3Pd) within the time interval following the minimum data value of the first derivative of the pressure curves (dPd) up to 15% of each cardiac cycle;
  - Mapping the data values obtained in the distal pressure curve (Pd);

- Calculating the systolic time period as the period between the beginning of each cardiac cycle and the calculated dicrotic notch;
- Calculating the diastolic period as the time period from the calculated dicrotic notch to the end of the cardiac cycle;
- Calculating the descent part of the diastolic curve (DPC) as the time period between the systolic peak, which is the first local maximum data value, and the end of the diastolic period;
- Calculating, for each cardiac cycle, the subpart of the curve (SPC) of the descent part of the diastolic curve (DPC), which is the time period between the first local minimum data value of the first derivative of the distal pressure curve (dPd) after the calculated dicrotic notch and the end of the diastolic period;
- Mapping the subpart of the curve (SPC) in the distal pressure curve (Pd);
- Calculating, for each cardiac cycle, the peak of the second derivative as the coronary stenosis indicator, in the following manner:

  - Applying a least square approach to provide the optimal degree polynomial approximation to the subpart of the curve (SPC) of each cardiac cycle of the distal pressure curve (Pd);

- Mapping the resulting optimal polynomial approximation in the distal pressure curve (Pd);
- Calculating the second derivative of the polynomial approximation of the distal pressure curve (d2Pd_Approx) using analytical differentiation rules;
- Calculating, for each cardiac cycle, the m maximum data values of the second derivative of the distal pressure curve (d2Pd_Approx), where *m* is a value between 1 and the total number of values in the subpart of the calculated curve;
- Calculating the arithmetic average (Av) of the m maximum data values;
- Calculating the median value (M) of Av values in the second derivative of the distal pressure curve (d2Pd_Approx) for 3 to 8 cardiac cycles;
- Calculating a cutoff value (C) from at least 200 distal pressure curve acquisitions classified as positive and negative diagnosis;
- Comparing the median value (M) with the cutoff value (C) in the following manner:
- If M > 1.05 x C, the indicator is considered positive;
- If M < 0.95 x C, the indicator is considered negative.

In one embodiment, the unfiltered distal pressure curve (unfPd) downstream of a stenosis is acquired at rest, or under partial hyperemia, or under total hyperemia conditions.

In one embodiment, the unfiltered distal pressure curve (unfPd) filtering is carried out by using a second order Savitzky-Golay filter.

In one embodiment, the distal pressure curve derivatives are obtained by using higher order numerical differentiation formulas.

In one embodiment, the dicrotic notch is calculated in the forthcoming 16% of each cardiac cycle.

In one embodiment, the polynomial approximation of the subpart of the curve (SPC) is calculated as: a degree n and a polynomial $p_n$ minimizing

$$\min_{n} \left( \min_{p_n} \sum_{i=1}^{k} \|(P_d)_i - p_n(t_i)\|^2 \right)$$

is found; where $t_i$ is the time instant at which the data value $(P_d)_i$ is measured.

In one embodiment, the cutoff value (C) is calculated to maximize the Youden Index:

$$Y = \mathrm{Se} + \mathrm{Sp} - 1$$

where the sensitivity Se is given by

$$\mathrm{Se} = \frac{TP}{TP + FN}$$

and the specificity Sp is given by

$$\mathrm{Sp} = \frac{TN}{TN + FP}$$

and the value *TP* corresponds to cases considered as True Positives, for which M > 1.05 x C, in agreement with a standard positive iFR test; *FP* represents the False Positives, as given by the iFR test; and the value *TN* corresponds to cases for which M < 0.95 x C in agreement with a standard negative iFR test.

**General description**

Coronary physiology assessment tests are used to assess the severity of a coronary lesion, particularly to evaluate the physiological severity of angiographically intermediate coronary stenotic lesions. FFR, iFR and cFFR are the current indicators used in clinical practice, that quantify the ratio between the pressure distal and proximal to a stenosis in predefined zones of the pressure curve recordings. In the present application it is proposed that the severity of a coronary lesion can be related with the geometry of the pressure curve distal to the stenosis.

A computer implemented method was developed where pressure measurements are analysed, and a new geometric indicator is automatically calculated, i.e. computed, in different zones of the cardiac cycle. The results obtained suggest that the geometrical behaviour of the distal pressure curve (Pd) is correlated with the severity of coronary lesions, as compared with established indicators such as FFR, iFR, cFFR. This finding has clinical applicability.

The present application relates to a computer implemented method for the assessment of the severity of a coronary stenosis at rest or under hyperaemia (whether induced by contrast or by a vasodilating agent) based on the measurement of the distal pressure curve concavity with a specifically calculated indicator.

The presently disclosed method has the following advantages:

- Integrated assessment of one indicator at rest or with different hyperaemia stimuli;
- Dedicated analysis of contrast hyperaemia;
- Simple method, without the need for adaptations of the usual practice in hemodynamics laboratories, which allows to quickly obtain the new indicator;
- Concave curve analysis - unprecedented method of physiological evaluation;
- Reduction in the occurrence of error due to deviation inherent in the reading of absolute values, as the shape is analysed, leading to greater reproducibility;
- In addition to its immediate clinical applicability, this method also has the potential to be used as an important research tool in the study of microvascular function, or in the study of the effects of contrast-induced hyperaemia.

**Brief description of drawings**

For easier understanding of this application, figures are attached in the annex that represent the preferred forms of implementation which nevertheless are not intended to limit the technique disclosed herein.

Figure 1 shows the distal pressure curve (Pd) (Figure 1 (a)) after the application of a filter; the first derivative of the filtered distal pressure curve (dPd) (Figure 1 (b)); and the second derivative of the filtered distal pressure curve (d2Pd) (Figure 1 (c));

Figure 2 shows the distal pressure curve (Figure 2 (a)) without the application of a filter (unfPd); the first derivative of the unfiltered distal pressure curve (unfdPd) (Figure 2 (b)); and the second derivative of the unfiltered distal pressure curve (unfd2Pd) (Figure 2 (c));

Figure 3 shows the first derivative of a filtered distal pressure curve (dPd) wherein the minimum data value of each cardiac cycle is identified with squared markers (■), the maximum data value of each cardiac cycle is identified with cross markers (x) and the zero data value of each cardiac cycle is identified with circular markers (•);

Figure 4 shows the minimum data value, maximum data value and zero data value of each cardiac cycle that were identified in the first derivative (Figure 3) mapped in the filtered distal pressure curve (Pd);

Figure 5(a) shows the mapped filtered distal pressure curve (Pd) of the dicrotic notch data value (•) of each cardiac cycle as identified in the first derivative (Figure 5(b)) or in the third derivative (Figure 5(c)) of the filtered distal pressure curve;

Figure 5(b) shows the dicrotic notch data value (•) of each cardiac cycle identified in the first derivative of the filtered distal pressure curve (dPd);

Figure 5(c) shows the dicrotic notch data value (•) of each cardiac cycle identified in the third derivative of the filtered distal pressure curve (d3Pd);

Figure 6 shows the descent part of the curve (DPC) in a filtered distal pressure curve (Pd).

Figure 7(a) shows the mapping, on the filtered distal pressure curve (Pd), of the value of the dicrotic notch data (•) and the subpart of the curve (SPC) of each cardiac cycle, as identified in the first derivative of the distal pressure curve (dPd) (Figure 7(b));

Figure 7(b) shows the value of the dicrotic notch data (•) and the subpart of the curve (SPC) of each cardiac cycle identified in the first derivative of the distal pressure curve (dPd);

Figure 8(a) shows the filtered distal pressure curve (Pd) and the mapping of the corresponding local polynomial approximation (Pd_Approx);

Figure 8(b) shows the second derivative of the filtered distal pressure curve (d2Pd) together with the second analytical derivative (d2Pd_Approx) of the local polynomial approximation (Pd_Approx) shown in Figure 8(a);

Figure 9 shows the overlap of the Pd and Pa curves for an example of a positive result;

Figure 10(a) shows the filtered distal pressure curve (Pd) and the corresponding local polynomial approximation (Pd_Approx), for an example of a negative result;

Figure 10(b) shows the second derivative of the filtered distal pressure curve (d2Pd) together with the second analytical derivative (d2Pd_Approx) of the local polynomial approximation (Pd_Approx) shown in Figure 11(a);

Figure 11 shows the overlap of the Pd and Pa curves for an example of a negative result.

## Description of embodiments

Now, preferred embodiments of the present application will be described in detail with reference to the annexed drawings. However, they are not intended to limit the scope of this application.

The present application relates to a computer implemented method configured to identify and calculate derivatives and data values of distal pressure curves (Pd) acquired from subjects (i.e., patients), under different conditions, in order to evaluate the severity of a coronary stenotic lesion.

The analysis of a distal pressure curve (Pd) mentioned above encompasses the assessment in three possible situations:

- rest, that is, without administration of any substance; or
- partial hyperemia, induced by intracoronary contrast (for example via an automatic injector or manually); or
- total hyperemia, induced by microcirculating vasodilator intravenous agent (adenosine or equivalent), as needed.

The presently disclosed computer implemented method allows:

- the identification of the beginning and end of each cardiac cycle based on the distal pressure curve (Pd), detection of relative cyclic minimum data values, associated with the beginning of a consistent increase in pressure [systolic upstroke], for defining the beginning and end of each cycle);
- filtering the data contained in the Pd curve;
- the calculation of the diastolic concavity.

In what follows, we refer to the mapping, in a given curve, of a quantity obtained at a certain time as the representation of the point in that curve which shares the same x coordinate (time carriable).

The computer implemented method of the present invention is performed as follows.

### 1) Data acquisition

The procedure requires the acquisition of blood pressure downstream of a previously identified stenosis in a subject, which is referred to as unfiltered distal pressure curve (unfPd). This acquisition is done using standard intracoronary catheterization.

In a non-limiting embodiment, the procedure initiates with the equalization of pressures with the catheter filled with blood (after allowing backflow of blood in the catheter) or saline solution. Special care is taken to avoid damping of pressures, with the equalization taking place in the ascending aorta if necessary. An intracoronary injection of nitrates (between 1 and 3 mg of Isosorbide dinitrate) is administered to prevent coronary vasospasm and produce a vasodilation of larger coronary arteries. The pressure wire is then advanced until the pressure sensor is two to three vessel diameter lengths distally to the lesion and Pd values are registered (at rest, under contrast-induced partial hyperaemia and/or under total hyperaemia with a vasodilating agent such as adenosine).

### 2) Data filtering

Due to the presence of noise inherent to the recording of a biological signal, the blood pressure recordings data (unfiltered distal pressure curve (unfPd)) is filtered. For each data value of the unfiltered distal pressure curve (unfPd), a regular function is fitted to all data values within a fixed interval of data values. The filtered value of signal on said data value is then obtained as the regular function value at that position. In one embodiment, an interval of 9 to 17 data values may be considered, according with the origin of the data.

An implementation of such filter can be done, for example, with the well-known second order Savitzky-Golay filter. This filtering technique reduces the noise while maintaining the shape and height of waveform peaks.

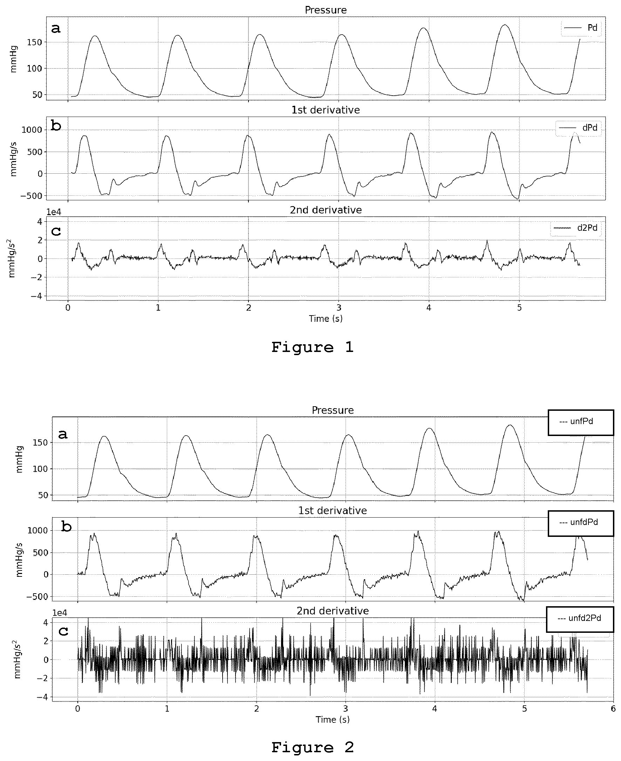

The resulting filtered data is depicted in Figure 1. They can be compared with the original unfiltered data depicted on Figure 2.

The filtered distal pressure curve is hereafter referred to only by 'distal pressure curve (Pd)'.

## 3) Calculation of curve's derivatives

The derivatives of different orders are calculated using higher order numerical differentiation formulas, based on the filtered distal pressure curve (Pd) data obtained previously. A possible option, for example, are the fourth order formulas to approximate the derivatives of a function $f$ at a discrete time data value $t_i$ given by:

$$f'(t_i) \approx \frac{-f_{i+2} + 8f_{i+1} - 8f_{i-1} + f_{i-2}}{12h}$$

$$f''(t) \approx \frac{-f_{i+2} + 16f_{i+1} - 30\ f_i + 16\ f_{i-1} - f_{i-2}}{12h^2}$$

$$f'''(t_i) \approx \frac{-f_{i+3} + 8f_{i+2} - 13f_{i+1} + 13\ f_{i-1} - 8\ f_{i-2} + f_{i-3}}{8h^3}$$

Where $f_{i+k} = f(t_i + kh)$, with $k$ an integer and $h$ a constant step given by $h = t - t_{i-1}$.

The first and second order derivatives of the distal pressure curve (Pd) of Figure 1(a) can be seen in Figure 1(b) and 1(c), respectively. For illustration purposes, the corresponding derivatives of the unfiltered original data are represented in Figures 2(b) and 2(c).

## 4) Identifying the curve zones of interest

### 4.1) Cardiac Cycles identification

The cardiac period T is the inverse of the cardiac frequency and is calculated as the average of the time intervals between consecutive systolic peaks, identified numerically from the distal pressure curve (Pd) data (Figure 1(a)). Within this period of time, the minimum value of the first derivative of the distal pressure curve (dPd) is identified.

The first derivative is calculated using higher order numerical differentiation formulas as disclosed above.

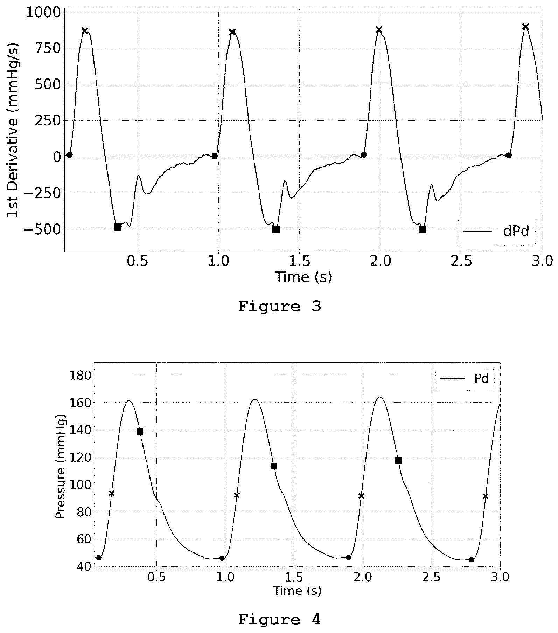

Figure 3 shows the first derivative of the distal pressure curve (dPd), and the minimum data value of each cardiac cycle is identified with squared markers (■), corresponding to a certain time t_min. The maximum data value of each cardiac cycle is also identified. This maximum data value should be looked for within the time interval [t_min, t_min+T]. These maxima data values are illustrated in Figure 3 using crossed markers (x).

The beginning of each cardiac cycle is defined as the zero of the first derivative, located between the minimum and the maximum data values of each cardiac cycle. In Figure 3, the zeros of the first derivative, for several cardiac cycles, are represented using circular markers (•). Also, in Figure 4, these zero data values, maxima and minima data values for each cardiac cycle, are identified when mapped in the distal pressure curve (Pd). The beginning of each cardiac cycle is then clearly identified (circular markers).

### 4.2) Dicrotic Notch

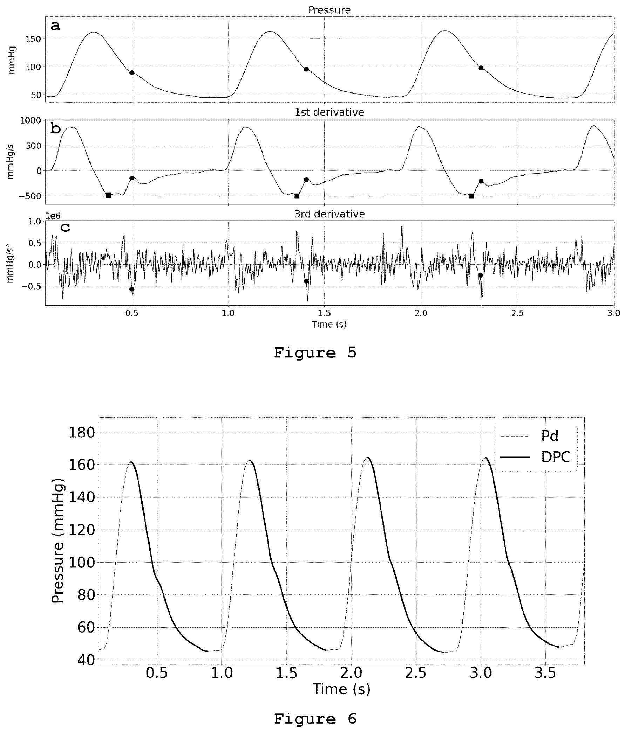

For each cardiac cycle previously identified in the distal pressure curve (Pd), the minimum data value (■) of the first derivative (Figure 5(b)) along the time interval defined by the first 50% to 70% of each cycle, preferably 60%, is identified.

For each cardiac cycle, and within the time interval beginning at the instant corresponding to this minimum and corresponding in length to 10 to 20 % of the cardiac cycle, preferably 16 % of each cardiac cycle, the data value closest to zero pressure (mmHg/s) is identified and referred to as 'zero'. Figure 5(b) shows this data value depicted with a circular marker (•) in the first derivative of the distal pressure curve (dPd) and then mapped in the distal pressure curve (Pd) by identifying the correspondence for the same time data value in the distal pressure curve (Pd), Figure 5(a). This zero is defined as the approximation of the dicrotic notch.

In cases where the waveform exhibits an incisure rather than a defined dicrotic notch, an alternative approach must be considered. In these situations, the incisure is defined as the location of the minimum data value of each cardiac cycle of the third derivative of the distal pressure curve (d3Pd) within the time interval following the minimum data value of the first derivative up to 15% of each cardiac cycle as shown in Figure 5(c).

### 4.3) Diastole Identification

The period comprised between the beginning of the cardiac cycle (Section 4.1) and the dicrotic notch (Section 4.2) is known as the systolic period. Afterwards, the diastolic period starts. The diastolic period is therefore defined as the time interval from the dicrotic notch to the end of the cardiac cycle, eventually discarding small oscillations that typically occur in the final 30% of the cycle.

The descent part of the diastolic curve (DPC) is the period between the systolic peak (first local maximum data value after its start) and the end of the diastole, as shown in Figure 6.

### 4.3.1) Subpart of the diastolic curve (SPC)

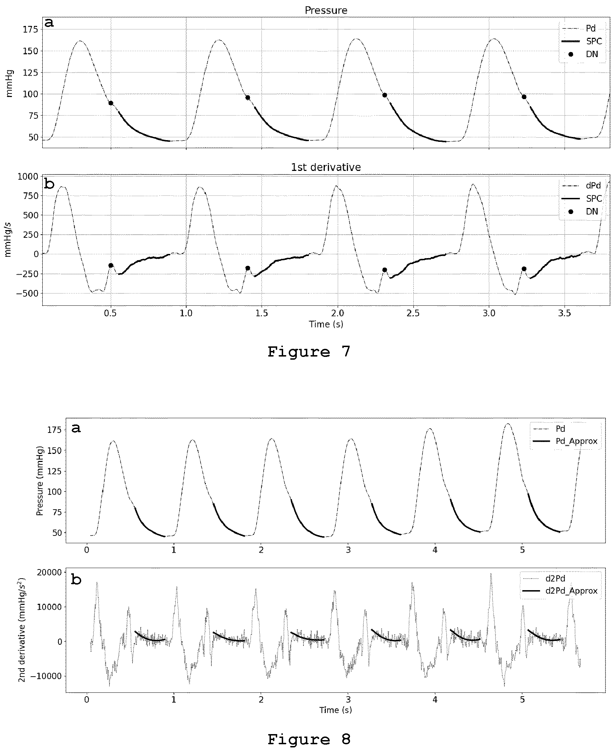

For each cardiac cycle, the subpart of the curve (SPC) of the descent part of the diastolic curve is identified as the period comprised between the first local minimum data value of the first derivative (dPd) after the dicrotic notch identified previously (see Figure 5) and the end of the diastole (see Figure 6). Figure 7(b) shows the SPC identified in the first derivative of the distal pressure curve (dPd) and Figure 7(a) shows the identified SPC mapped in the distal pressure curve (Pd).

### 5) Indicator calculation

### Calculating the peak of the second derivative

### - Polynomial approximation of the subpart of the curve (SPC)

For each cycle, a least squares approach is used to provide the optimal degree polynomial approximation for the distal pressure curve (Pd) data in the subpart of the curve (SPC). Specifically, a degree n and a polynomial $p_n$ minimizing

$$\min_n \left( \min_{p_n} \sum_{i=1}^{k} \| (P_d)_i - p_n(t_i) \|^2 \right)$$

is found. Here $t_i$ is the time instant at which the data value $(P_d)_i$ was measured, $n$ is the degree of the polynomials fitting the Pd curve at $k$ time steps.

Figure 8(a) shows the resulting optimal polynomial approximation mapped in the distal pressure curve (Pd) data.

It is important to find the polynomial that approximates the most to the SPC of each cardiac cycle, this polynomial approximation allows to obtain the second derivative (d2Pd_Approx) and a smoother graphical representation of the data.

### - Calculation of the second derivative on the subpart of the curve (SPC)

The second derivative of the polynomial approximation (d2Pd_Approx) is calculated directly using analytical differentiation rules.

In Figure 8(b), the corresponding polynomial second derivative (d2Pd_Approx) can be seen overlapped with the oscillating second derivative calculated (d2Pd) from the distal pressure curve (Pd), using a higher order numerical approximation.

This approach eliminates the high frequency oscillations still present in the second derivative (d2Pd), when calculated directly from the distal pressure curve (Pd).

**- Average maximum data values of the second derivative**

The m maximum data values, wherein m is a value between 1 and the total number of values on the SPC, are identified in the second derivative (d2Pd_Approx) of each cardiac cycle, and their arithmetic average (Av) is calculated. This value provides an indicator of the curve concavity.

**- Median data values of the second derivative**

The median value (M) of the Av values identified in the second derivative (d2Pd_Approx) for 3 to 8 cardiac cycles, preferable 5 cycles, is calculated. This statistical measure of central tendency is calculated in order to minimize the effect of possible outliers.

The comparison with a cutoff value (C) will determine the outcome of the test and allow to assess the severity of the stenosis of a subject:

If M > 1.05 x C the indicator is considered positive.
If M < 0.95 x C, the indicator is considered negative.
Otherwise, the result is inconclusive.

**- *A priori* determination of the cutoff (C)**

*A priori* determination of (C) requires the use of at least 200 pressure curve acquisitions classified as positive and negative diagnosis, for example, using the known indicator iFR.

The cutoff value C is calculated as to maximize the Youden Index:

$$Y = Se + Sp - 1$$

where the sensitivity Se is given by

$$Se = \frac{TP}{TP + FN}$$

and the specificity Sp is given by

$$Sp = \frac{TN}{TN + FP}$$

The value *TP* corresponds to cases considered as True Positives, for which M > 1.05 x C, in agreement with a standard positive iFR test (iFR < 0.86).

Wherein a positive iFR test means that this lesion would likely benefit from revascularization.

Accordingly, *FP* represents the False Positives, as given by the iFR test.

The *TN* value corresponds to True Negatives, meaning, cases where M < 0.95 x C in agreement with a standard negative iFR test (iFR > 0.93).

Wherein a negative iFR test means that this lesion would probably benefit from medical therapy alone, without revascularization.

Similarly, *FN* stands for the False Negatives.

Thus, the presently disclosed computer implemented method for the assessment of the severity of a coronary stenosis comprises the following steps:

- Acquiring an unfiltered distal pressure curve (unfPd) downstream of a stenosis previously identified in a subject;
- Filtering the unfiltered the distal pressure curve data (unfPd) by fitting a regular function to the data to obtain a filtered distal pressure curve (Pd);
- Identifying, in the previously filtered distal pressure curve (Pd), the cardiac cycles within a cardiac time period T, which is calculated as the average of the time intervals between consecutive systolic peaks;
- Calculating the first derivative of the pressure curve (dPd) and calculating for each cardiac cycle:

  - the minimum data value corresponding to a t_min time;

- the maximum data value in a time interval [t_min, t_min+T];
- the zero data value corresponding to the beginning of each cardiac cycle and located between the minimum and the maximum data values;
- Mapping the data values in the distal pressure curve (Pd);

- Calculating the dicrotic notch for each cardiac cycle of the distal pressure curve (Pd) by using the first derivative of the pressure curve (dPd) to calculate:

  - The minimum data value along the first 50 to 70% time interval of each cardiac cycle;
  - The dicrotic notch as being the zero data value closest to zero pressure in the forthcoming 10 to 20% time interval of each cardiac cycle; or identifying the zero data values in the third derivative of the pressure curves (d3Pd) within the time interval following the minimum data value of the first derivative of the pressure curves (dPd) up to 15% of each cardiac cycle;
  - Mapping the data values obtained in the distal pressure curve (Pd);

- Calculating the systolic time period as the period between the beginning of each cardiac cycle and the calculated dicrotic notch;
- Calculating the diastolic period as the time period from the calculated dicrotic notch to the end of the cardiac cycle;
- Calculating the descent part of the diastolic curve (DPC) as the time period between the systolic peak, which is the first local maximum data value, and the end of the diastolic period;
- Calculating, for each cardiac cycle, the subpart of the curve (SPC) of the descent part of the diastolic curve (DPC), which is the time period between the first local minimum data value of the first derivative of the distal pressure curve (dPd) after the calculated dicrotic notch and the end of the diastolic period;
- Mapping the subpart of the curve (SPC) in the distal pressure curve (Pd);
- Calculating, for each cardiac cycle, the peak of the second derivative as the coronary stenosis indicator, in the following manner:

  - Applying a least square approach to provide the optimal degree polynomial approximation to the subpart of the curve (SPC) of each cardiac cycle of the distal pressure curve (Pd);
  - Mapping the resulting optimal polynomial approximation in the distal pressure curve (Pd);
  - Calculating the second derivative of the polynomial approximation of the distal pressure curve (d2Pd_Approx) using analytical differentiation rules;
  - Calculating, for each cardiac cycle, the m maximum data values of the second derivative of the distal pressure curve (d2Pd_Approx), where $m$ is a value between 1 and the total number of values in the subpart of the calculated curve;
  - Calculating the arithmetic average (Av) of the $m$ maximum data values;
  - Calculating the median value (M) of Av values in the second derivative of the distal pressure curve (d2Pd_Approx) for 3 to 8 cardiac cycles;
  - Calculating a cutoff value (C) from at least 200 distal pressure curve acquisitions classified as positive and negative diagnosis;
  - Comparing the median value (M) with the cutoff value (C) in the following manner:

    - If $M > 1.05 \times C$, the indicator is considered positive;
    - If $M < 0.95 \times C$, the indicator is considered negative.

**Example:**

Using a data base of 208 pressure measurements associated with iFR validated test results, a cutoff C = 1983 was obtained and calculated in order to maximize the Youden Index, as described above.

iFR, an index commonly used to assess whether a stenosis is causing a limitation of blood flow in coronary arteries with subsequent ischemia, was used as comparison to access the validity of the data values obtained with the method of the present invention.

**Positive outcome from the peak of the second derivative according to the present invention**

In the case of the distal pressure curve (Pd) represented in Figure 8(a), the measured iFR was 0.74, which indicates the lesion is hemodynamically significant.

The calculated peak of the second derivative, obtained as described above, was $M = 3367 > 1.05 \times 1983$ giving a

positive outcome, as expected.

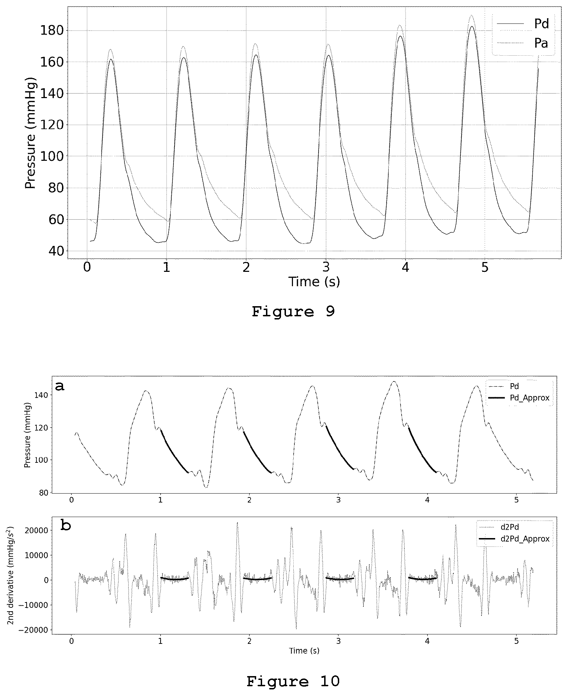

The curves of the distal pressure curve (Pd) and the aortic pressure (Pa) can be seen in Figure 9, and one can observe the difference in absolute values in the diastolic period, giving the positive iFR value. The intensity of the pressure curve concavity is contained on each of the second derivatives of the local approximation shown in Figure 8(b).

This information is captured by the concavity indicator M = 3367 - a positive result.

In practice, this means the patients will likely benefit from revascularization.

## Negative outcome from the peak of the second derivative according to the present invention

In the case of the distal pressure curve (Pd) represented in Figure 10(a), the measured iFR was 0.96.

The calculated peak of the second derivative, obtained as described above, was M = 876 < 0.95 x 1983 giving a negative outcome, as expected.

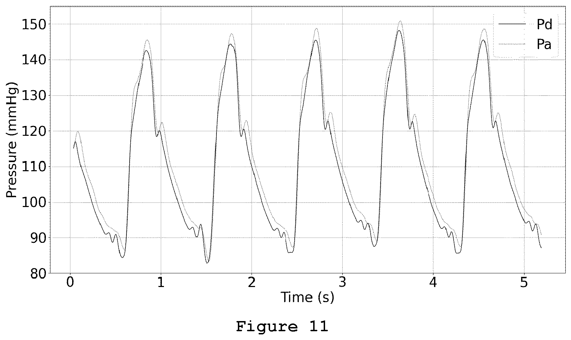

The curves of the distal pressure curve (Pd) and the aortic pressure (Pa) can be seen in Figure 11, and one can observe the similarity in absolute values in the diastolic period, giving the negative iFR value.

The low intensity of the pressure curve concavity is contained on each of the second derivatives of the local approximation shown in Figure 10(b). This information is captured by the low concavity indicator M = 876 - a negative result.

In practice, this means the patients will likely benefit from medical therapy only, without revascularization.

This description is of course not in any way restricted to the forms of implementation presented herein and any person with an average knowledge of the area can provide many possibilities for modification thereof without departing from the general idea as defined by the claims. The preferred forms of implementation described above can obviously be combined with each other. The following claims further define the preferred forms of implementation.

## Bibliography

[1] P. Libby and P. Theroux, "Pathophysiology of Coronary Artery Disease The Pathophysiology of Chronic CAD," Circulation, vol. 111, pp. 3481-3488, 2005.

[2] J. Knuuti, W. Wijns, A. Saraste, E. Barbato, D. Capodanno, C. Funck-brentano, E. Prescott, R. F. Storey, C. Deaton, T. Cuisset, S. Agewall, K. Dickstein, T. Edvardsen, J. Escaned, B. J. Gersh, P. Svitil, M. Gilard, D. Hasdai, R. Hatala, F. Mahfoud, J. Masip, C. Muneretto, M. Valgimigli, S. Achenbach, and J. J. Bax, "2019 ESC Guidelines for the diagnosis and management of chronic coronary syndromes," European Heart Journal, vol. 00, pp. 7-21, 2019.

[3] E. J. Topol and P. S. Teirstein, Textbook of Interventional Cardiology, 7th ed. Elsevier, 2016.

[4] M. Roffi, C. Patrono, J. P. Collet, C. Mueller, M. Valgimigli, F. Andreotti, J. J. Bax, M. A. Borger, C. Brotons, D. P. Chew, B. Gencer, G. Hasenfuss, K. Kjeldsen, P. Lancellotti, U. Landmesser, J. Mehilli, D. Mukherjee, R. F. Storey, and S. Windecker, "2015 ESC Guidelines for the management of acute coronary syndromes in patients presenting without persistent st-segment elevation: Task force for the management of acute coronary syndromes in patients presenting without persistent ST-segment elevation," European Heart Journal, vol. 37, no. 3, pp. 273-278, 2016.

[5] N. H. Pijls, J. A. Van Son, R. L. Kirkeeide, B. De Bruyne, and K. L. Gould, "Experimental basis of determining maximum coronary, myocardial, and collateral blood flow by pressure measurements for assessing functional stenosis severity before and after percutaneous transluminal coronary angioplasty" Circulation, vol. 86, no. 4, pp. 1354-1367, 1993.

[6] J. Z. Lee, N. Singh, I. Nyotowidjojo, C. Howe, S.-W. Low, T. Nguyen, D. Pinto, G. Kumar, and K. S. Lee, "Comparison of regadenoson and nitroprusside to adenosine for measurement of fractional flow reserve: A systematic review and meta-analysis," Cardiovascular Revascularization Medicine, vol. 19, no. 2, pp. 168-174, mar 2018.

[7] G. Crystal and L. Klein, "Fractional Flow Reserve: Physiological Basis, Advantages and Limitations, and Potential Gender Differences," Current Cardiology Reviews, vol. 11, no. 3, pp. 209-219, 2015.

[8] S. Sen, J. Escaned, I. S. Malik, G. W. Mikhail, R. A. Foale, R. Mila, J. Tarkin, R. Petraco, C. Broyd, R. Jabbour, A. Sethi, C. S. Baker, M. Bellamy, M. Al-Bustami, D. Hackett, M. Khan, D. Lefroy, K. H. Parker, A. D. Hughes, D. P. Francis, C. Di Mario, J. Mayet, and J. E. Davies, "Development and Validation of a New Adenosine-Independent Index of Stenosis Severity From Coronary Wave-Intensity Analysis," Journal of the American College of Cardiology, vol. 59, no. 15, pp. 1392-1402, apr 2012.

[9] S. V. Guzman and J. W. West, "Cardiac effects of intracoronary arterial injections of various roentgenographic contrast media," American Heart Journal, vol. 58, no. 4, pp. 597-607, oct 1959.

[10] A. M. Leone, G. Scalone, G. L. De Maria, F. Tagliaferro, A. Gardi, F. Clemente, E. Basile, P. Cialdella, A. R. De Caterina, I. Porto, C. Aurigemma, F. Burzotta, G. Niccoli, C. Trani, A. G. Rebuzzi, and F. Crea, "Efficacy of contrast medium induced Pd/Pa ratio in predicting functional significance of intermediate coronary artery stenosis assessed by fractional flow reserve: insights from the RINASCI study," EuroIntervention, vol. 11, no. 4, pp. 421-427, aug 2015.
[11] S. Shah and S. Pfau, "Coronary Physiology in the Cardiac Catheterization Laboratory," Journal of Clinical Medicine, vol. 8, no. 2, p. 255, feb 2019.

**Claims**

1. Computer implemented method for the assessment of the severity of a coronary stenosis according to the following steps:

   - Acquiring an unfiltered distal pressure curve (unfPd) downstream of a stenosis previously identified in a subject;
   - Filtering the unfiltered the distal pressure curve data (unfPd) by fitting a regular function to the data to obtain a filtered distal pressure curve (Pd);
   - Identifying, in the previously filtered distal pressure curve (Pd), the cardiac cycles within a cardiac time period T, which is calculated as the average of the time intervals between consecutive systolic peaks;
   - Calculating the first derivative of the pressure curve (dPd) and calculating for each cardiac cycle:

       - the minimum data value corresponding to a t_min time;
       - the maximum data value in a time interval [t_min, t_min+T];
       - the zero data value corresponding to the beginning of each cardiac cycle and located between the minimum and the maximum data values;
       - Mapping the data values in the distal pressure curve (Pd);

   - Calculating the dicrotic notch for each cardiac cycle of the distal pressure curve (Pd) by using the first derivative of the pressure curve (dPd) to calculate:

       - The minimum data value along the first 50 to 70% time interval of each cardiac cycle;
       - The dicrotic notch as being the zero data value closest to zero pressure in the forthcoming 10 to 20% time interval of each cardiac cycle; or identifying the zero data values in the third derivative of the pressure curves (d3Pd) within the time interval following the minimum data value of the first derivative of the pressure curves (dPd) up to 15% of each cardiac cycle;
       - Mapping the data values obtained in the distal pressure curve (Pd);

   - Calculating the systolic time period as the period between the beginning of each cardiac cycle and the calculated dicrotic notch;
   - Calculating the diastolic period as the time period from the calculated dicrotic notch to the end of the cardiac cycle;
   - Calculating the descent part of the diastolic curve (DPC) as the time period between the systolic peak, which is the first local maximum data value, and the end of the diastolic period;
   - Calculating, for each cardiac cycle, the subpart of the curve (SPC) of the descent part of the diastolic curve (DPC), which is the time period between the first local minimum data value of the first derivative of the distal pressure curve (dPd) after the calculated dicrotic notch and the end of the diastolic period;
   - Mapping the subpart of the curve (SPC) in the distal pressure curve (Pd);
   - Calculating, for each cardiac cycle, the peak of the second derivative as the coronary stenosis indicator, in the following manner:

       - Applying a least square approach to provide the optimal degree polynomial approximation to the subpart of the curve (SPC) of each cardiac cycle of the distal pressure curve (Pd);
       - Mapping the resulting optimal polynomial approximation in the distal pressure curve (Pd);

   - Calculating the second derivative of the polynomial approximation of the distal pressure curve (d2Pd_Approx) using analytical differentiation rules;
   - Calculating, for each cardiac cycle, the m maximum data values of the second derivative of the distal pressure curve (d2Pd_Approx), where $m$ is a value between 1 and the total number of values in the subpart of the calculated curve;
   - Calculating the arithmetic average (Av) of the m maximum data values;
   - Calculating the median value (M) of Av values in the second derivative of the distal pressure curve (d2Pd_Approx) for 3 to 8 cardiac cycles;

- Calculating a cutoff value (C) from at least 200 distal pressure curve acquisitions classified as positive and negative diagnosis;
- Comparing the median value (M) with the cutoff value (C) in the following manner:

     - If M > 1.05 x C, the indicator is considered positive;
     - If M < 0.95 x C, the indicator is considered negative.

2. Method according to the previous claim, wherein the unfiltered distal pressure curve (unfPd) downstream of a stenosis is acquired at rest, or under partial hyperemia, or under total hyperemia conditions.

3. Method according to any of the previous claims, wherein the unfiltered distal pressure curve (unfPd) filtering is carried out by using a second order Savitzky-Golay filter.

4. Method according to any of the previous claims, wherein the distal pressure curve derivatives are obtained by using higher order numerical differentiation formulas.

5. Method according to any of the previous claims, wherein the dicrotic notch is calculated in the forthcoming 16% of each cardiac cycle.

6. Method according to any of the previous claims, wherein the polynomial approximation of the subpart of the curve (SPC) is calculated as: a degree n and a polynomial $p_n$ minimizing

$$\min_{n}\left( \min_{p_n} \sum_{i=1}^{k} \|(P_d)_i - p_n(t_i)\|^2 \right)$$

is found; where $t_i$ is the time instant at which the data value $(P_d)_i$ is measured.

7. Method according to any of the previous claims, wherein the cutoff value (C) is calculated to maximize the Youden Index:

$$Y = \text{Se} + \text{Sp} - 1$$

where the sensitivity Se is given by

$$\text{Se} = \frac{TP}{TP + FN}$$

and the specificity Sp is given by

$$\text{Sp} = \frac{TN}{TN + FP}$$

and the value *TP* corresponds to cases considered as True Positives, for which M > 1.05 x C, in agreement with a standard positive iFR test; FP represents the False Positives, as given by the iFR test; and the value *TN* corresponds to cases for which M < 0.95 x C in agreement with a standard negative iFR test.

Figure 1

Figure 2

Figure 3

Figure 4

Figure 5

Figure 6

Figure 7

Figure 8

Figure 9

Figure 10

Figure 11

| | Europäisches Patentamt European Patent Office Office européen des brevets | **EUROPEAN SEARCH REPORT** | **Application Number** EP 25 17 6864 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2019/320911 A1 (SUH JUNG W [KR] ET AL) 24 October 2019 (2019-10-24) <br> * paragraph [0006] * <br> * paragraph [0008] - paragraph [0012] * <br> * paragraph [0038] - paragraph [0049] * <br> * paragraph [0053] * <br> * paragraph [0055] * <br> * paragraph [0084] - paragraph [0086] * <br> * paragraph [0098] * <br> * paragraph [0110] * <br> * paragraph [0120] * <br> * figures 3A,3B,4-9 * <br> ----- | 1-7 | INV. A61B5/02 A61B5/0215 A61B5/00 |
| A | SAYAN SEN ET AL: "Development and Validation of a New Adenosine-Independent Index of Stenosis Severity From Coronary Wave-Intensity Analysis", JOURNAL OF THE AMERICAN COLLEGE OF CARDIOLOGY, vol. 59, no. 15, 1 April 2012 (2012-04-01) , pages 1392-1402, XP055173991, ISSN: 0735-1097, DOI: 10.1016/j.jacc.2011.11.003 <br> * page 1394, left-hand column, last paragraph - page 1396, right-hand column, paragraph 1 * <br> ----- | 1-7 | |
| | | | TECHNICAL FIELDS SEARCHED (IPC) A61B |
| A | US 2021/251496 A1 (DAVIES JUSTIN [GB] ET AL) 19 August 2021 (2021-08-19) <br> * paragraph [0007] - paragraph [0008] * <br> * paragraph [0057] * <br> * paragraph [0074] - paragraph [0075] * <br> * paragraph [0083] - paragraph [0084] * <br> * paragraph [0088] - paragraph [0089] * <br> * figures 2,3,9-18 * <br> ----- <br> -/-- | 1-7 | |

The present search report has been drawn up for all claims

| Place of search | Date of completion of the search | Examiner |
|---|---|---|
| Berlin | 5 September 2025 | Völlinger, Martin |

EPO FORM 1503 03.82 (P04C01)

page 1 of 2

# EUROPEAN SEARCH REPORT

**Application Number**

EP 25 17 6864

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2022/354371 A1 (WARNER ADRIAN F [US] ET AL) 10 November 2022 (2022-11-10)<br>* paragraph [0003] *<br>* paragraph [0006] *<br>* paragraph [0021] *<br>* paragraph [0027] - paragraph [0031] *<br>* paragraph [0045] - paragraph [0046] *<br>* figures 2-7 *<br>----- | 1-7 | |

TECHNICAL FIELDS
SEARCHED     (IPC)

The present search report has been drawn up for all claims

| Place of search | Date of completion of the search | Examiner |
|---|---|---|
| Berlin | 5 September 2025 | Völlinger, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

## ANNEX TO THE EUROPEAN SEARCH REPORT
## ON EUROPEAN PATENT APPLICATION NO.

EP 25 17 6864

This annex lists the patent family members relating to the patent documents cited in the above-mentioned European search report.
The members are as contained in the European Patent Office EDP file on
The European Patent Office is in no way liable for these particulars which are merely given for the purpose of information.

05-09-2025

| Patent document cited in search report | | Publication date | Patent family member(s) | | Publication date |
|---|---|---|---|---|---|
| US 2019320911 | A1 | 24-10-2019 | CN | 111954487 A | 17-11-2020 |
| | | | EP | 3781026 A1 | 24-02-2021 |
| | | | ES | 2915828 T3 | 27-06-2022 |
| | | | JP | 7671318 B2 | 01-05-2025 |
| | | | JP | 2021521943 A | 30-08-2021 |
| | | | JP | 2023126224 A | 07-09-2023 |
| | | | PL | 3781026 T3 | 11-07-2022 |
| | | | US | 2019320911 A1 | 24-10-2019 |
| | | | US | 2020329981 A1 | 22-10-2020 |
| | | | WO | 2019203895 A1 | 24-10-2019 |
| US 2021251496 | A1 | 19-08-2021 | CA | 2846607 A1 | 28-02-2013 |
| | | | EP | 2744401 A2 | 25-06-2014 |
| | | | EP | 3120762 A1 | 25-01-2017 |
| | | | EP | 3581102 A1 | 18-12-2019 |
| | | | EP | 3581103 A1 | 18-12-2019 |
| | | | EP | 3685738 A1 | 29-07-2020 |
| | | | ES | 2599373 T3 | 01-02-2017 |
| | | | ES | 2782900 T3 | 16-09-2020 |
| | | | JP | 6129174 B2 | 17-05-2017 |
| | | | JP | 2014529442 A | 13-11-2014 |
| | | | JP | 2017504415 A | 09-02-2017 |
| | | | RU | 2014110701 A | 27-09-2015 |
| | | | US | 2013046190 A1 | 21-02-2013 |
| | | | US | 2014207008 A1 | 24-07-2014 |
| | | | US | 2016206214 A1 | 21-07-2016 |
| | | | US | 2021251496 A1 | 19-08-2021 |
| | | | US | 2024245303 A1 | 25-07-2024 |
| | | | WO | 2013028613 A2 | 28-02-2013 |
| US 2022354371 | A1 | 10-11-2022 | CN | 110638432 A | 03-01-2020 |
| | | | EP | 3586729 A1 | 01-01-2020 |
| | | | JP | 7034986 B2 | 14-03-2022 |
| | | | JP | 2020011054 A | 23-01-2020 |
| | | | US | 2019387981 A1 | 26-12-2019 |
| | | | US | 2022354371 A1 | 10-11-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030032886 A, Dgany E
- US 20140276137 A1, Burnett J

**Non-patent literature cited in the description**

- **P. LIBBY** ; **P. THEROUX**. Pathophysiology of Coronary Artery Disease The Pathophysiology of Chronic CAD. *Circulation*, 2005, vol. 111, 3481-3488
- **J. KNUUTI** ; **W. WIJNS** ; **A. SARASTE** ; **E. BARBATO** ; **D. CAPODANNO** ; **C. FUNCK-BREN-TANO** ; **E. PRESCOTT** ; **R. F. STOREY** ; **C. DEATON** ; **T. CUISSET**. 2019 ESC Guidelines for the diagnosis and management of chronic coronary syndromes. *European Heart Journal*, 2019, vol. 00, 7-21
- **E. J. TOPOL** ; **P. S. TEIRSTEIN**. Textbook of Interventional Cardiology. Elsevier, 2016
- **M. ROFFI** ; **C. PATRONO** ; **J. P. COLLET** ; **C. MUELLER** ; **M. VALGIMIGLI** ; **F. ANDREOTTI** ; **J. J. BAX** ; **M. A. BORGER** ; **C. BROTONS** ; **D. P. CHEW**. 2015 ESC Guidelines for the management of acute coronary syndromes in patients presenting without persistent st-segment elevation: Task force for the management of acute coronary syndromes in patients presenting without persistent ST-segment elevation. *European Heart Journal*, 2016, vol. 37 (3), 273-278
- **N. H. PIJLS** ; **J. A. VAN SON** ; **R. L. KIRKEEIDE** ; **B. DE BRUYNE** ; **K. L. GOULD**. Experimental basis of determining maximum coronary, myocardial, and collateral blood flow by pressure measurements for assessing functional stenosis severity before and after percutaneous transluminal coronary angioplasty. *Circulation*, 1993, vol. 86 (4), 1354-1367

- **J. Z. LEE** ; **N. SINGH** ; **I. NYOTOWIDJOJO** ; **C. HOWE** ; **S.-W. LOW** ; **T. NGUYEN** ; **D. PINTO** ; **G. KUMAR** ; **K. S. LEE**. Comparison of regadenoson and nitroprusside to adenosine for measurement of fractional flow reserve: A systematic review and meta-analysis. *Cardiovascular Revascularization Medicine*, March 2018, vol. 19 (2), 168-174
- **G. CRYSTAL** ; **L. KLEIN**. Fractional Flow Reserve: Physiological Basis, Advantages and Limitations, and Potential Gender Differences. *Current Cardiology Reviews*, 2015, vol. 11 (3), 209-219
- **S. SEN** ; **J. ESCANED** ; **I. S. MALIK** ; **G. W. MIKHAIL** ; **R. A. FOALE** ; **R. MILA** ; **J. TARKIN** ; **R. PETRACO** ; **C. BROYD** ; **R. JABBOUR**. Development and Validation of a New Adenosine-Independent Index of Stenosis Severity From Coronary Wave-Intensity Analysis. *Journal of the American College of Cardiology*, April 2012, vol. 59 (15), 1392-1402
- **S. V. GUZMAN** ; **J. W. WEST**. Cardiac effects of intracoronary arterial injections of various roentgenographic contrast media. *American Heart Journal*, October 1959, vol. 58 (4), 597-607
- **A. M. LEONE** ; **G. SCALONE** ; **G. L. DE MARIA** ; **F. TAGLIAFERRO** ; **A. GARDI** ; **F. CLEMENTE** ; **E. BASILE** ; **P. CIALDELLA** ; **A. R. DE CATERINA** ; **I. PORTO**. Efficacy of contrast medium induced Pd/Pa ratio in predicting functional significance of intermediate coronary artery stenosis assessed by fractional flow reserve: insights from the RINASCI study. *EuroIntervention*, August 2015, vol. 11 (4), 421-427
- **S. SHAH** ; **S. PFAU**. Coronary Physiology in the Cardiac Catheterization Laboratory. *Journal of Clinical Medicine*, February 2019, vol. 8 (2), 255